# EUROPÄISCHE PATENTSCHRIFT

(11) **EP 2 653 177 B1**
(45) Veröffentlichungstag und Bekanntmachung des Hinweises auf die Patenterteilung: **12.11.2014**
(21) Anmeldenummer: 13160455.5
(22) Anmeldetag: 21.03.2013
(51) Int. Cl.: A61M 1/14, A61M 1/36

(54) **Vorrichtung zur extrakorporalen Blutbehandlung mit Leckagesensor**
Apparatus for extracorporeal blood processing with leakage sensor
Appareil pour traitement extracorporel du sang avec détecteur de fuite

(30) Priorität: 20.04.2012 DE 102012103504
(43) Veröffentlichungstag der Anmeldung: 23.10.2013
(73) Patentinhaber: B. Braun Avitum AG, 34212 Melsungen (DE)
(72) Erfinder: Bröker, Björn, 34355 Staufenberg (DE)
(74) Vertreter: Winter, Brandl, Fürniss, Hübner, Röss, Kaiser, Polte - Partnerschaft mbB

(56) Entgegenhaltungen:
- WO-A1-2004/096322
- WO-A1-2009/090473
- DE-A1-102009 051 993
- US-A1- 2003 126 910
- US-A1- 2011 315 237
- US-A1- 2011 315 611

## Beschreibung

Die Erfindung betrifft eine Vorrichtung zur extrakorporalen Blutbehandlung, wenigstens umfassend einen extrakorporalen Blutkreislauf zum Pumpen von Blut. Die Vorrichtung kann ferner einen Dialysator für den Stoffaustausch zwischen dem Blut und einem Dialysat und ein Hydrauliksystem zur Bereitstellung des Dialysats umfassen.

Bei solchen Dialysegeräten können Leckagen im extrakorporalen Schlauchsystem oder in der Verschlauchung im Bilanzierungskreislauf bei Auftreten während der Therapie zu Ultrafiltrationsabweichungen und Blutverlust führen, was unter Umständen schwerwiegende Folgen für den Patienten haben kann. Der Dichtigkeitstest des Hydraulikkreislaufes und des extrakorporalen Blutkreislaufes wird bei den meisten Geräten einmalig oder zyklisch durch einen Drucktest realisiert, eine kontinuierliche Überwachung während aller Therapiephasen gestaltet sich jedoch schwierig. Daher werden zur Feststellung von Leckagen oftmals entsprechende Sensoren eingesetzt.

Da es mehrere mögliche Ursachen für Undichtigkeiten geben kann, sind üblicherweise mehrere Sensoren erforderlich, um die einzelnen Leckagen festzustellen. Beispielsweise ist es bekannt, insgesamt drei Sensoren zur Detektion von Leckagen einzusetzen, wobei sich jeweils ein Sensor im Geräteinneren, im Gehäuse der DF-Filter und in der Auffangrinne für Flüssigkeiten auf einer Frontplatte befindet.

Es ist jedoch auch möglich, alle möglicherweise anfallenden Leckageflüssigkeiten aus unterschiedlichen Quellen in einem Sammelbehälter im unteren Bereich des Dialysegeräts aufzufangen und nur einen Sensor vorzusehen, der dann den Füllstand des Sammelbehälters misst und zum Beispiel ab einem bestimmten Füllstand einen Alarm auslöst. Die DE 196 05 260 B4 beschreibt beispielsweise einen Leckagesensor auf dem wannenförmigen Boden eines Dialysegeräts. Fluide wie Blut, Wasser oder Dialysat, die über Lecks aus verschiedenen Modulen fließen können, werden in einem Sammelbecken am Boden des Geräts gesammelt. Derartige Leckströme können auf beliebigen Wegen fließen. Der Boden des Gehäuses des Geräts ist dabei horizontal nicht eben, damit das Sammeln des Fluids erleichtert wird, wie es etwa von der Ölwanne eines Motors her bekannt ist. Der Boden des Geräts kann die Form einer Schüssel oder irgendeinen anderen geeigneten Umriss haben, der ein unteres Sammelbecken bereitstellt. Ein Fluidsensor ist dann in der Nähe des Sammelbeckens angeordnet, um das Auftreten von Fluid im Sammelbecken anzuzeigen. Wenn durch den Sensor Fluid detektiert wird, löst das Betriebssystem bzw. Schutzsystem einen Alarm aus, um den Anwender über ein akustisches oder visuelles Signal zu alarmieren, und das Gerät wird auf Lecks überprüft. Die US 5,674,390 offenbart eine ähnliche Ausführungsform einer Auffangwanne, bei welcher ein Sensor an der tiefsten Stelle der Wanne angebracht ist.

Ferner offenbart die WO 2004/096322 A1 ein Dialysegerät mit einer trichterförmigen Auffangwanne im Bodenbereich, in der offensichtlich alle Leckageflüssigkeiten zusammenlaufen und an der zwei Sensoren auf unterschiedlichen Höhen angebracht sind. Der untere Sensor detektiert dann eine untere Füllstandsgrenze an Leckageflüssigkeiten, während der zweite Sensor einen höheren Füllstand misst. Darüber hinaus beschreibt die WO 2009/090473 A1 einen Leckagesensor, der aus dem Geräteinneren nach außen verlagert ist.

Weitere Vorrichtungen zur extrakorporalen Blutbehandlung, die im Bodenbereich Auffangwannen zur Aufnahme von Leckageflüssigkeiten aufweisen, sind aus der US 2011/0315611 A1, der US 2003/0126910 A1 und der US 2011/0315237 A1 bekannt.

Wird keine Rinne auf einer Frontplatte verwendet, sondern eine zentrale Auffangmulde in dem Sockel des Geräts, können auch Tropfen von den Konzentratkanistern zu dem jeweiligen Sensor gelangen. Daher ist eine zu hohe Empfindlichkeit der verwendeten Sensoren kontraproduktiv, da diese ansonsten lediglich aufgrund von bestimmten Mengen an Konzentrat, die noch keine Gefährdung darstellen, auslösen würden, ohne dass ein Leck im Gerät vorliegt, denn diese Konzentrattropfen könnten beispielsweise auch beim Anschluss von Kanistern an das Dialysegerät auftreten.

Im Falle von Leckagen muss die Flüssigkeit zudem auch wieder entfernt werden, so dass Auffangbehälter vorzugsweise leicht zugänglich sein sollten, damit Flüssigkeiten vom Personal leicht entfernt werden können.

Aufgabe der Erfindung ist es daher, eine Vorrichtung zur extrakorporalen Blutbehandlung bereitzustellen, bei der Leckageflüssigkeiten aus unterschiedlichen Quellen sicher detektiert werden können, wobei jedoch geringe Mengen an sichtbaren Leckageflüssigkeiten, die keine Gefährdung darstellen, leicht entfernbar sein sollen, bevor ein Alarm ausgelöst wird.

Erfindungsgemäß wird diese Aufgabe durch eine Vorrichtung gemäß dem unabhängigen Anspruch 1 gelöst. Vorteilhafte Weiterbildungen der Vorrichtung ergeben sich aus den Unteransprüchen 2-16.

Die erfindungsgemäße Vorrichtung zur extrakorporalen Blutbehandlung umfasst wenigstens einen extrakorporalen Blutkreislauf zum Pumpen von Blut. Die einzelnen Komponenten dieses extrakorporalen Blutkreislaufs befinden sich dabei jeweils innerhalb und außerhalb eines Gehäuses der Vorrichtung, wobei unterhalb des Gehäuses ein Sockel angebracht ist, der eine Auffangmulde aufweist, in der innerhalb des Gehäuses entstehende Leckageflüssigkeiten auffangbar sind. Die Vorrichtung weist einen Sensor auf, mit dem wenigstens das Vorhandensein von Flüssigkeit innerhalb der Auffangmulde messbar ist. Erfindungsgemäß weist die Vorrichtung ferner Aufnahmemittel zur Aufnahme von außerhalb des Gehäuses entstehenden Leckageflüssigkeiten auf, wobei diese Aufnahmemittel so ausgebildet sind, dass sie die außerhalb des Gehäuses entstehenden Leckageflüssigkeiten ab einer vorgegebenen Menge wenigstens teilweise in die Auffangmulde überführen.

Dieser Aufbau führt dazu, dass Leckageflüssigkeiten, welche durch Komponenten im Inneren der Vorrichtung entstehen, direkt in einer Auffangmulde gesammelt werden, und ein zugehöriger Sensor ist so ausgeführt, dass er das Vorhandensein von Flüssigkeit in der Auffangmulde detektieren kann. Dieses Signal kann entsprechend durch eine Steuer- und Auswerteeinheit ausgewertet werden, indem beispielsweise ein Alarm erzeugt und die Behandlung unterbrochen wird. Ergänzend werden Leckageflüssigkeiten, die außerhalb des Gehäuses entstehen, nur verzögert in die Auffangmulde überführt. Sie würden daher erst ab einer bestimmten Menge zum Auslösen eines Alarms führen. Die erfindungsgemäße Vorrichtung zur extrakorporalen Blutbehandlung differenziert somit zwischen internen Leckagen, die ohne Verzögerung in die Auffangwanne zum Sensor geleitet werden und externen Leckagen, die beispielsweise erst sichtbar auf dem Sockel gesammelt werden, bevor sie zum Sensor gelangen können. Dadurch ist gewährleistet, dass durch den Sensor nur relevanten Flüssigkeitsmengen detektiert werden, dabei jedoch trotz unterschiedlicher Empfindlichkeitsanforderungen nur ein Sensor erforderlich ist.

Dies hat den Vorteil, dass ungefährliche Mengen an Leckageflüssigkeit vorher vom Bedienpersonal entfernt werden könnten, ohne dass es zu einem Alarm kommen muss. Vorzugsweise sind die Aufnahmemittel, in denen die außerhalb des Gehäuses entstehenden Flüssigkeiten bis zu einem bestimmten Mengenwert gesammelt werden, dazu von außerhalb des Gehäuses sichtbar und zugänglich. So kann das Bedienpersonal sehen, dass sich externe Leckageflüssigkeiten sammeln und kann diese beispielsweise einfach wegwischen, ohne das die Therapie durch einen Alarm gestört würde. Fällt dem Bedienpersonal jedoch nicht auf, dass sich Leckageflüssigkeiten von außen liegenden Komponenten sammeln, oder handelt es sich um so große Mengen, dass von einem größeren Leck auszugehen ist, würden diese in die Auffangmulde überführt. Die Steuereinheit, die das Sensorsignal empfängt, wird dann einen Alarm auslösen und die Behandlung unterbrechen.

Typischerweise umfasst die Vorrichtung zur extrakorporalen Blutbehandlung neben dem extrakorporalen Blutkreislauf zum Pumpen von Blut auch ein Hydrauliksystem zur Bereitstellung von Dialysierflüssigkeit, wobei sich einzelne Komponenten des Hydrauliksystems ebenfalls innerhalb und außerhalb des Gehäuses der Vorrichtung befinden können. Beispielsweise umfasst das Hydrauliksystem wenigstens einen Vorratsbehälter bzw. Kanister, der ein Medium zur Zubereitung des Dialysats enthält. Wenigstens dieser Vorratsbehälter kann sich dabei außerhalb des Gehäuses befinden, aber auch Schlauchleitungen des Hydrauliksystems können außerhalb des Gehäuses angeordnet sein. Oftmals kommt es dann vor, dass sich an solchen Schlauchleitungen und Vorratsbehältern beim Konnektieren Tropfen bilden, die an der Frontseite des Gehäuses heruntertropfen oder herunterfließen.

Das Blutschlauchsystem wird typischerweise auf der Vorderseite des Geräts montiert und zum Vorbereiten der Behandlung mit Flüssigkeit gefüllt. Auch an den Konnektionsstellen des Blutschlauchsystems können sich Tropfen bilden und an der Frontplatte des Geräts herunterlaufen. Insbesondere diese können bei kleineren Mengen einfach bei der üblichen Oberflächenreinigung des Geräts abgewischt werden.

In einer Ausführungsform der Erfindung erstreckt sich der Sockel der Vorrichtung wenigstens an der Frontseite des Gehäuses über das Gehäuse hinaus. In diesem Frontbereich des Sockels, der dann von außen sichtbar und zugänglich ist, können sowohl Teile der Auffangmulde, als auch der Sensor und/oder die Aufnahmemittel zur verzögerten Zuführung von äußeren Leckageflüssigkeiten in die Auffangmulde angeordnet sein. Zweckmäßigerweise befindet sich der Sensor im unteren Bereich der Auffangmulde.

Als Aufnahmemittel kann in dem außerhalb des Gehäuses liegenden Frontbereich des Sockels beispielsweise wenigstens eine nach oben offene Rinne vorgesehen sein, die eine Verbindung zur Auffangmulde aufweist, wobei die Verbindung zwischen der Rinne und der Auffangmulde so ausgebildet ist, dass in der Rinne gesammelte Leckageflüssigkeiten nur ab einer vorgegebenen Menge in die Auffangmulde fließen. Das Volumen der Rinne bzw. die Verbindung zur Auffangmulde sind dabei entsprechend gewählt, um die gewünschte Verzögerung zu erreichen. Die Verbindung zwischen der Rinne und der Auffangmulde kann auf verschiedene Arten realisiert werden, wobei sich ein Steg zwischen der Rinne und der Auffangmulde als einfaches und effektives Mittel erwiesen hat. Die Höhe des Stegs liegt dann unterhalb des Rands der Rinne, so dass sich Flüssigkeit in der Rinne so lange sammelt, bis der Pegel die Oberkante des Stegs erreicht. Dann fließt so viel Flüssigkeit über den Steg in die Auffangmulde, dass der Pegel wieder genau an der Oberkante des Stegs liegt.

In dem beschriebenen Ausführungsbeispiel mit einer Rinne als Aufnahmemittel und einem Steg zur Volumenregulierung ergibt sich die vorgegebene Menge an Leckageflüssigkeit, bei welcher diese an die Auffangmulde überführt wird, somit aus dem Volumen an angesammelter Flüssigkeit. Die Menge kann jedoch auch anders definiert sein. Beispielsweise sind Ausführungsformen denkbar, bei denen sich extern entstandene Leckageflüssigkeiten so lange an oder auf einer Platte sammeln, bis ein bestimmtes Gewicht erreicht ist und die Platte sich absenkt oder kippt, um die Flüssigkeit dann an die Auffangmulde zu überführen. In diesem Fall wäre die relevante Menge für die Überführung der Flüssigkeit durch das Gewicht der Flüssigkeit definiert.

Ferner könnten statt der Sammlung von externen Leckageflüssigkeiten in einem Behälter auch andere Arten von Aufnahmemitteln eingesetzt werden, um die gewünschte Verzögerung zu erreichen. Beispielsweise wäre es möglich, externe Leckageflüssigkeiten erst in einem schwammartigen Material aufzufangen, das so gewählt ist, dass die Leckageflüssigkeit wieder aus dem Material heraustropft, wenn eine bestimmte Sättigung erreicht ist. Hierbei wäre die relevante Menge je nach Ausführungsform wieder über das Volumen bzw. das Gewicht der Flüssigkeit definiert. Manuell entfernt werden könnte die Flüssigkeit dann beispielsweise durch Auspressen des Schwammmaterials.

Eine Lösung mit einer Rinne im Sockel stellt somit zwar eine bevorzugte Ausführungsform der Erfindung dar, die Erfindung ist jedoch nicht auf diese Form von Aufnahmemitteln beschränkt. Auch die relevante Menge für die Überführung von Flüssigkeit in die Auffangmulde mit Sensor ist nicht auf das Volumen oder Gewicht der Flüssigkeit beschränkt, sondern richtet sich nach den gewählten Aufnahmemitteln. Die vorgegebene Menge an Flüssigkeit, bei der diese an die Auffangmulde überführt wird, kann auch frei wählbar sein, so dass sie beispielsweise vom Bedienpersonal eingestellt werden kann. Dies kann insbesondere durch bewegliche Bauteile erreicht werden, wenn beispielsweise der beschriebene Steg in der Höhe verändert werden kann.

In einem Ausführungsbeispiel der Erfindung weist der Sockel im Bereich außerhalb des Gehäuses ferner Mittel zur Zuführung von Leckageflüssigkeiten zu den Aufnahmemitteln auf. Hierbei kann es sich beispielsweise um entsprechend geneigte Flächen handeln, auf denen Tropfen in Richtung einer Rinne abfließen.

In einem weiteren Ausführungsbeispiel der Erfindung erstreckt sich die Auffangmulde ebenfalls über das Gehäuse hinaus, und der Sensor ist in diesem außerhalb des Gehäuses liegenden Bereich der Auffangmulde positioniert. Dies hat den Vorteil, dass sowohl die Auffangmulde, als auch der Sensor von außen zugänglich sind und einfach gereinigt werden können. Insbesondere bei einer Reinigung des Sensors ist kein Werkzeug erforderlich. Leckageflüssigkeiten aus dem Inneren des Gehäuses werden dann zwar im Inneren aufgefangen, aber dann nach außen zum Sensor geleitet, wenn dort der tiefste Punkt der Auffangmulde liegt. Hierbei muss jedoch durch eine entsprechende Ausformung des Sockels gewährleistet sein, dass nur Leckageflüssigkeiten aus dem Inneren des Gehäuses direkt in die Auffangmulde geleitet werden, während sich Leckageflüssigkeiten von der Frontseite des Geräts erst in den jeweils gewählten Aufnahmemitteln sammeln und nicht auch direkt in die Auffangmulde fließen. Dies kann beispielsweise dadurch erreicht werden, dass der außerhalb des Gehäuses liegende Bereich der Auffangmulde eine abnehmbare Abdeckung aufweist. So kann außen keine Flüssigkeit von oben in die Auffangmulde tropfen, aber das Bedienpersonal hat dennoch einfachen Zugriff auf die Auffangmulde und den Sensor, wenn es die Abdeckung bei Bedarf entfernt oder hochklappt.

In einem Ausführungsbeispiel der Erfindung ist das Gehäuse ferner so ausgebildet, dass außerhalb des Gehäuses entstehende Leckageflüssigkeiten aufgrund einer Kapillarwirkung zu den Aufnahmemitteln geleitet werden. Hierzu kann die Oberfläche der Frontseite des Gehäuses beispielsweise mehrere eingeprägte Rillen aufweisen.

Darüber hinaus kann der Sensor und/oder eine an den Sensor angeschlossene Auswerteinheit der Vorrichtung so ausgebildet sein, dass bei der Erkennung von Flüssigkeiten in der Auffangmulde spezifisch zwischen zwei oder mehr Flüssigkeiten aus der Gruppe Blut, Dialyseflüssigkeit, Konzentrat, Desinfektionsflüssigkeit und Wasser unterschieden werden kann. Beispielsweise kann diese Unterscheidung der Flüssigkeiten farbselektiv durchführbar sein.

Ferner ist vorzugsweise vorgesehen, dass der extrakorporale Blutkreislauf ein Blutschlauchsystem und das Hydrauliksystem ein Dialyseflüssigkeits-Flusssystem umfasst, und dass die Vorrichtung eine Steuer- und/oder Kontrolleinheit aufweist, durch welche bei Erkennung von Leckagen mittels des Sensors eine automatische Dichtigkeitsüberprüfung des Dialyseflüssigkeits-Flusssystems und/oder des Blutschlauchsystems initiierbar ist.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung der Komponenten eines Dialysegeräts;
- Fig. 2: eine dreidimensionale Ansicht eines Ausführungsbeispiels eines Sockels mit Leckagesensor;
- Fig. 3: einen Längsschnitt durch einen Sockel gemäß Fig. 2;
- Fig. 4: eine dreidimensionale Teilansicht eines Sockels mit Abdeckung;
- Fig. 5: eine schematische Darstellung von möglichen Quellen und der Handhabung von Leckageflüssigkeiten; und
- Fig. 6: ein Prinzipschaltbild des Dialyseflüssigkeitskreislaufs und des extrakorporalen Blutkreislaufs mit Steuerung und Anzeige.

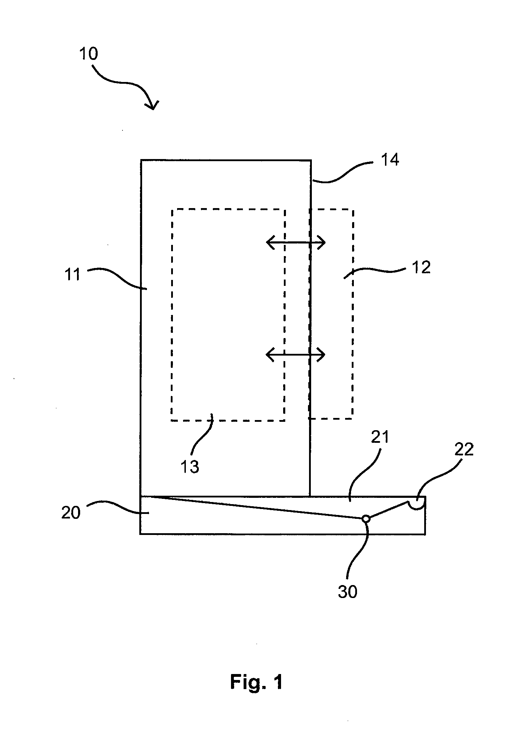

In Fig. 1 sind die für die Erfindung wesentlichen Komponenten bzw. Bereiche eines Dialysegeräts 10 schematisch dargestellt, wobei das Gerät eine Hämofiltration (HF), eine Hämodialyse (HD) oder eine Kombination beider Dialyseverfahren in Form einer Hämodiafiltration (HDF) durchführen kann. Dabei umfasst das Dialysegerät 10 wenigstens einen extrakorporalen Blutkreislauf zum Pumpen von Blut, einen Dialysator für den Stoffaustausch zwischen dem Blut und einem Dialysat und ein Hydrauliksystem zur Bereitstellung des Dialysats bzw. der Dialyselösung im Dialysator. Der Blutkreislauf besteht dabei aus einem Blutmodul des Dialysegeräts und einem Blut-schlauchsystem, das üblicherweise als Einmalartikel ausgeführt ist. Das Blutmodul kann wenigstens Zugabestellen für Medikamente, Druckmesser, eine Blutpumpe und Sensoren zur Erkennung von Luftblasen im Schlauchsystem umfassen. Der Dialysator weist eine semipermeable Membran auf, über welche der Stoffaustausch zwischen dem Blut und dem Dialysat erfolgt, wobei der Dialysator in den Blutkreislauf geschaltet ist.

Das Hydrauliksystem (Bilanzierungskreislauf) umfasst ebenfalls ein Schlauchsystem, Filter und wenigstens einen Vorratsbehälter, der ein Medium zur Zubereitung des Dialysats enthält. Dabei kann es sich beispielsweise um Wasser, Bicarbonat und/oder Laktat handeln. Dabei sind die einzelnen Komponenten des Blutkreislaufs und des Hydrauliksystems nicht im Detail dargestellt, sondern Fig. 1 zeigt lediglich schematisch, dass sich einige Komponenten bzw. Teile des extrakorporalen Blutkreislaufs und des Hydrauliksystems innerhalb des Gehäuses der Vorrichtung befinden, wobei diese Komponenten gestrichelt dargestellt sind und allgemein mit der Bezugsziffer 13 gekennzeichnet sind.

Andere Komponenten des extrakorporalen Blutkreislaufs und des Hydrauliksystems sind dagegen außerhalb des Gehäuses 11 angeordnet, was ebenfalls gestrichelt dargestellt und mit der Bezugsziffer 12 gekennzeichnet ist. Dabei befinden sich diese externen Komponenten insbesondere an der Frontseite 14 des Dialysegeräts 10. Typischerweise handelt es sich dabei um Schläuche und Anschlüsse, aber auch um Kanister, in denen sich Medien zur Bereitstellung des Dialysats befinden.

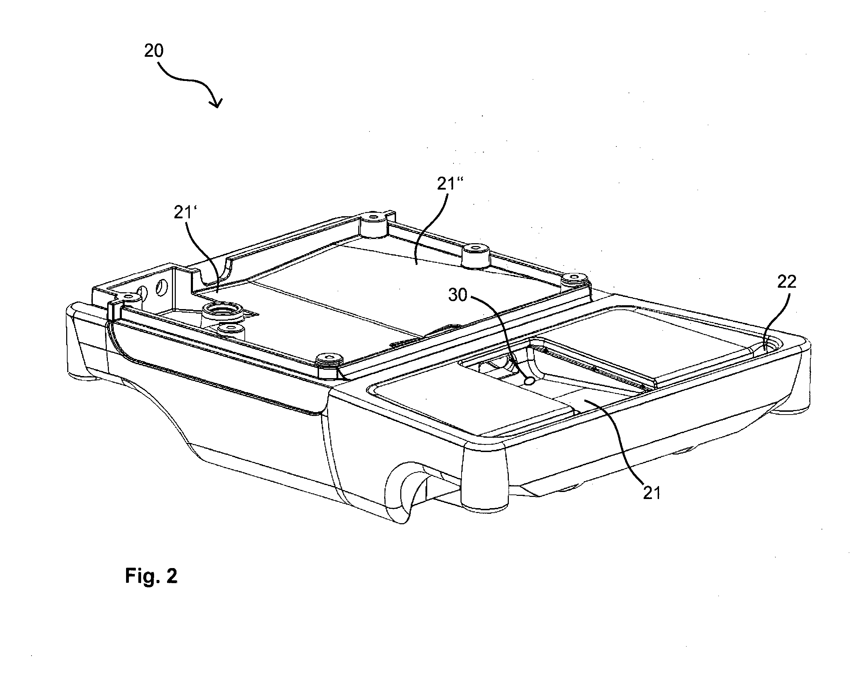

Unterhalb des Gehäuses 11 ist ein Sockel 20 angeordnet, in dem sich eine Auffangmulde 21 befindet. Der Sockel 20 kann aus Kunststoff bestehen und einstückig ausgeformt oder aus mehreren Teilen zusammengefügt sein. Die Auffangmulde 21 ist dabei so ausgeformt, dass Leckageflüssigkeiten, die aus den inneren Komponenten 13 des Dialysegeräts 10 austreten könnten, im Bodenbereich des Geräts 10 aufgefangen und gesammelt werden. In dem in Fig. 1 dargestellten Ausführungsbeispiel der Erfindung erstreckt sich der Sockel 20 zusammen mit der Auffangmulde 21 über das Gehäuse 11 hinaus. Vorzugsweise ist dies wenigstens an der Frontseite 14 des Geräts der Fall, so dass Leckageflüssigkeiten, die in diesem Frontbereich 14 durch die äußeren Komponenten 12 des Blutkreislaufs und/oder des Hydrauliksystems entstehen können, ebenfalls im Sockel 20 aufgefangen werden. Dazu ist am Rand des Sockels 20 eine Rinne 22 vorgesehen. Der Aufbau eines solchen Sockels 20 mit Rinne 22 ist in den Figuren 2 bis 4 im Detail gezeigt.

Fig. 2 zeigt ein Ausführungsbeispiel eines Sockels 20, bei dem sich der tiefste Punkt der Auffangmulde 21 außerhalb des nicht dargestellten Gehäuses 11 des Dialysegeräts 10 befindet. Die Auffangmulde 21 ist in diesem Fall nicht wie eine Mulde mit gleichmäßig konkav ausgeformten Muldenseiten ausgebildet, sondern weist innerhalb des Gehäuses zwei schräge Wannenflächen 21' und 21' auf, mit denen Flüssigkeiten, welche auf diese Wannenflächen tropfen, in die Mitte des Geräts und zur Frontseite 14 hin geleitet werden. Dort gelangen sie durch einen Luftspalt in den Bereich der Auffangmulde 21, in dem sich der Sensor 30 befindet. Die Auffangmulde 21 wird somit durch die Wannenflächen 21', 21" innerhalb des Gehäuses und die zugehörige Mulde außerhalb des Gehäuses gebildet. Der Sensor 30 ist je nach Bauform und Funktionsweise vorzugsweise so an der geformten Auffangmulde 21 angebracht, dass Flüssigkeit im unteren Bereich der Mulde detektiert wird. Dies ist in dem dargestellten Ausführungsbeispiel außerhalb des Gehäuses der Fall. Es kann jedoch auch vorgesehen sein, den tiefsten Punkt der Mulde 21 und damit den Sensor 30 unterhalb des Gehäuses vorzusehen. In diesem Fall wäre der Sensor zwar nicht mehr frei zugänglich, aber diese Ausführung könnte unter Umständen andere Vorteile mit sich bringen. Der Sensor 30 kann beispielsweise auf optischen Weg Flüssigkeit detektieren, es sind jedoch auch andere Sensoren wie Schwimmerschalter, elektrische Kontakte (Strommessung) oder Ultraschallstrecken denkbar.

Am Rand des äußeren Bereichs des Sockels 20 ist eine nach oben offene Rinne 22 ausgeformt, die quer am vorderen Rand des Sockels 20 verläuft oder anderweitig ausgebildet sein kann. Beispielsweise könnte eine U-förmige Rinne vorteilhaft sein, wenn diese am Rand des Sockels 20 entlang läuft und so jede Flüssigkeit auffängt, die nach außen abfließt. Zwischen dieser Rinne 22 und der Auffangmulde 21 ist der Sockel 20 daher vorzugsweise so ausgeführt, dass Flüssigkeiten, die durch Leckagen an den äußeren Komponenten des Dialysegeräts 10 entstehen, zur Rinne 22 hin geleitet werden und nicht direkt in die Auffangmulde 21 fließen. Dazu ist die Oberfläche des Sockels 20 außerhalb der Auffangmulde 21 und der Rinne 22 beispielsweise entsprechend schräg ausgebildet.

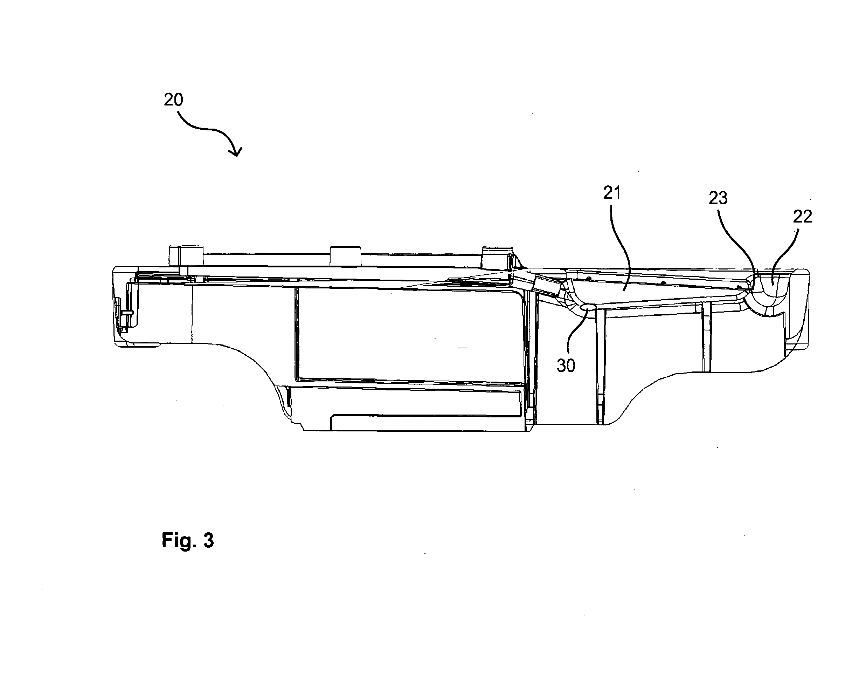

Durch diese Ausformung bzw. Konturierung des Sockels 20 sammeln sich die Leckageflüssigkeiten aus dem Inneren des Gehäuses direkt in der Auffangmulde 21, während sich mögliche Leckageflüssigkeiten aus den äußeren Komponenten des Geräts 10 erst in der Rinne 22 sammeln. Wie dem Schnitt in Fig. 3 zu entnehmen ist, ist zwischen der Rinne 22 und der Auffangmulde 21 ein Steg 23 angebracht, der eine Verbindung zwischen diesen beiden Auffangmitteln grundsätzlich verhindert. Allerdings liegt die Oberkante des Stegs 23 unterhalb des äußeren Rands der Rinne 22, so dass Flüssigkeiten, die sich in der Rinne 22 sammeln, oberhalb eines bestimmten Pegels über den Steg 23 in die Auffangmulde 21 fließen. Die Höhe des Stegs 23 kann dann zusammen mit dem Volumen des Rinne 22 so gewählt werden, dass externe Leckageflüssigkeiten ab einem bestimmten Volumen an die Auffangmulde 21 und damit den Sensor 30 überführt werden. Vorzugsweise ist dieses Volumen so gewählt, dass Flüssigkeit erst von der Rinne 22 in die Auffangmulde 21 und somit zum Sensor 30 gelangt, wenn sich in der Rinne 22 mehr Flüssigkeit gesammelt hat, als unter normalen Betriebsbedingungen aufgrund von Abwischen und/oder Tropfen, etc. zu erwarten ist.

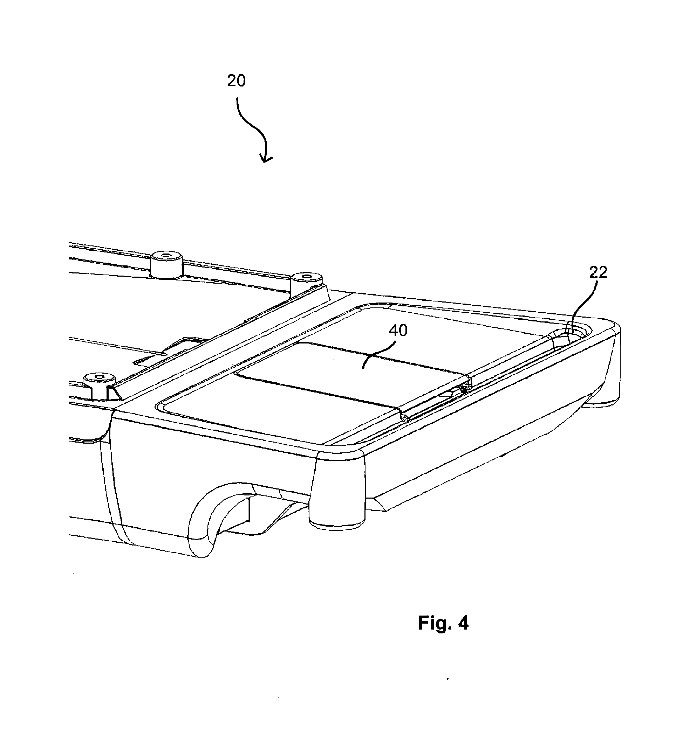

Fig. 4 zeigt den Sockel 20 in einer Ausführungsform, bei welcher die Auffangmulde mit einer abnehmbaren Abdeckung 40 verdeckt ist. So wird auch ein direktes Tropfen von Flüssigkeiten in die Auffangmulde 21 verhindert. Die Auffangmulde 21 und somit auch der Sensor 30 sind dennoch für das Bedienpersonal leicht erreichbar, so dass der Sensor 30 beispielsweise auch leicht und ohne Anwendung eines Werkzeugs gereinigt werden kann.

Durch den beschriebenen Aufbau des Sockels 20 gelangen Leckageflüssigkeiten aus den äußeren Komponenten 12 des Dialysegeräts 10 verzögert in die Auffangmulde 21, während sich Leckageflüssigkeiten aus den inneren Komponenten 13 direkt in der Auffangmulde 21 sammeln und vom Sensor 30 detektiert werden können. Dabei kann der Sensor 30 so ausgebildet bzw. an eine Auswerteeinheit angeschlossen sein, dass er bei Detektion einer Flüssigkeit in der Auffangmulde 21 sofort einen Alarm auslöst. Auf diese Weise würde das Bedienpersonal sofort darauf aufmerksam gemacht, wenn Flüssigkeit in der Auffangmulde 21 ankommt. Es kann jedoch auch vorgesehen sein, dass der Sensor 30 erst ab einem bestimmten Flüssigkeitspegel innerhalb der Auffangmulde 21 einen Alarm auslöst, oder es sind mehrere Pegelgrenzen programmiert, bei denen der Sensor unterschiedliche Alarme auslöst.

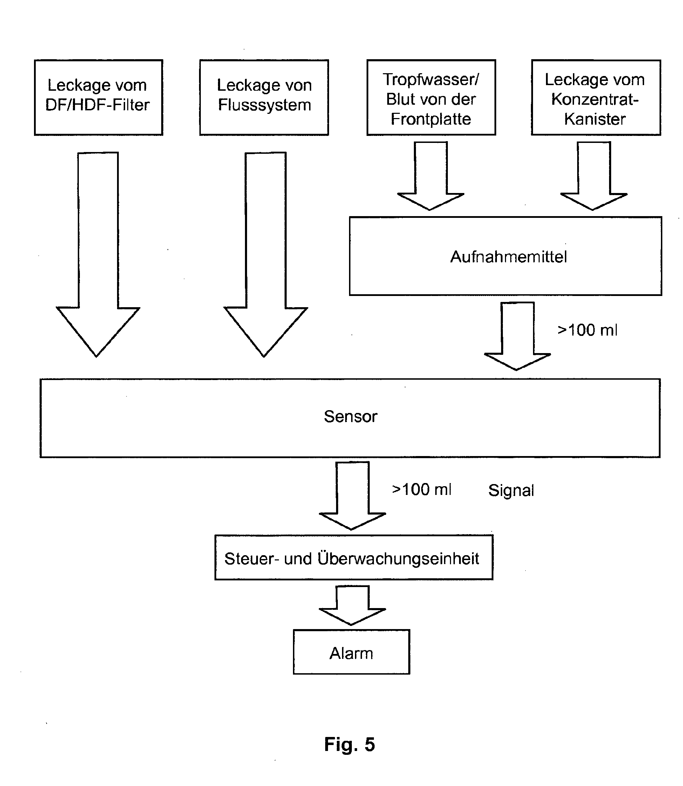

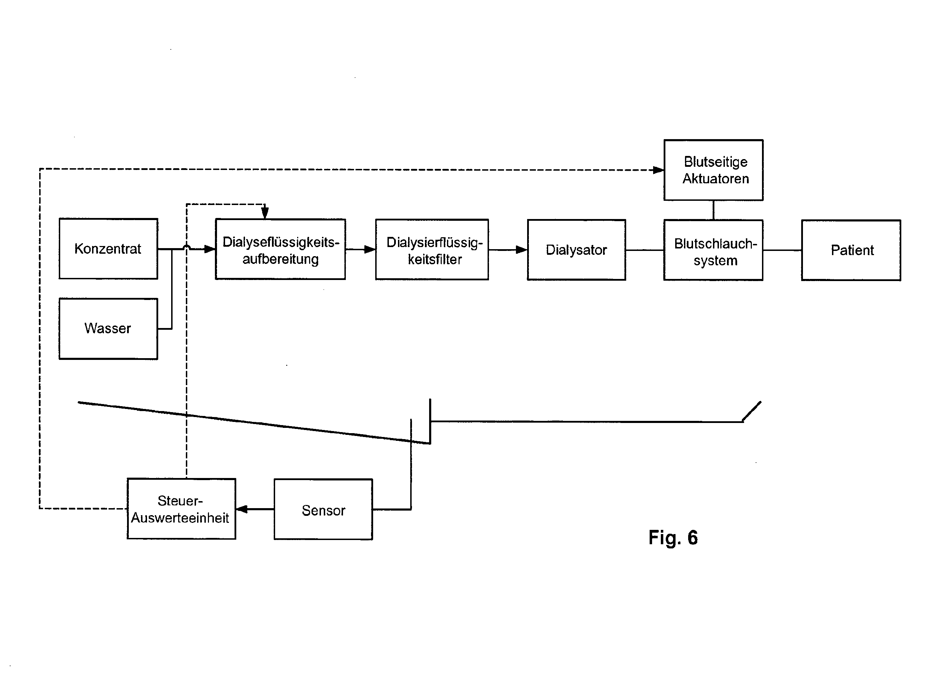

Fig. 5 zeigt schematisch in einem Diagramm die möglichen Quellen für Leckageflüssigkeiten und deren mögliche Handhabung durch die erfindungsgemäße Vorrichtung. Beispielsweise können Leckagen von DF/HDF-Filtern und/oder Leckagen im Dialyseflüssigkeits-Flusssystem direkt im Inneren des Geräts an den Sensor geleitet werden, während Tropfwasser oder Blut vom Frontpanel und/oder Flüssigkeiten von den Kanistern zur Dialysatbereitstellung erst einem Aufnahmemittel zugeführt werden. Erst ab beispielsweise einer Menge von 100ml werden diese Flüssigkeiten dann ebenfalls an den Sensor weitergeleitet. Dieser löst dann ebenfalls erst ab einer Gesamtmenge an Flüssigkeiten von 100ml einen Alarm aus. Fig. 6 zeigt dazu ein Prinzipschaltbild des Dialyseflüssigkeitskreislaufs und des extrakorporalen Blutkreislaufs mit einer Steuerung und einer Anzeige.

Die Steuer- und Auswerteeinheit des Dialysegeräts reagiert auf die Signale des Sensors 30 bei Vorhandensein von Flüssigkeit beispielsweise durch Initiierung eines entsprechenden Hinweises an den Bediener, falls das Gerät die Ursache der Flüssigkeit nicht erkennen kann. Es können jedoch auch Überprüfungsmechanismen vorgesehen sein, um die Ursache der Leckage zu ermitteln und dem Bediener anzuzeigen. Wenn der Anwender die Flüssigkeit durch Auswischen und/oder Aufsaugen entfernt und dabei sicher ist, dass die Ursache kein Gerätedefekt ist, kann die Behandlung fortgesetzt werden. Denkbar wäre auch das Starten eines automatischen Dichtigkeitstests des Hydraulikkreislaufs und/oder des Blutschlauchsystems (Druckhaltetest), um die korrekte Funktion des Geräts zu überprüfen.

In einem Ausführungsbeispiel der Erfindung ist der Sensor 30 als optischer Sensor ausgeführt, der eine Flüssigkeit anhand optischer Grenzflächenreflektion detektiert. Auch optoelektronische Sensoren können verwendet werden. Alternativ kann der Sensor auch auf kapazitiver Basis realisiert werden und zugleich die Auffangmulde 21 bilden.

In einem Ausführungsbeispiel der Erfindung können darüber hinaus farbselektive Sensoren eingesetzt werden. So kann beispielsweise spezifisch zwischen verschiedenen Leckageflüssigkeiten wie Wasser oder Blut unterschieden werden, wodurch verschiedene Schutzziele wie Ultrafiltrationsabweichungen und Blutverluste unterschiedlich priorisiert werden könnten.

Die Leckageflüssigkeiten können ferner durch eine bestimmte Gestaltung des Gehäuses 11 zu den verschiedenen Auffangbehältern geleitet werden. Insbesondere die Frontseite 14 des Gehäuses 11 kann beispielsweise so ausgebildet sein, dass durch Kapillarwirkung eine gerichtete Flüssigkeitsführung erfolgt. Dazu kann die Oberfläche der Frontseite 14 des Gehäuses 11 mehrere eingeprägte Rillen aufweisen, oder die Spalten des Gehäuses 11 haben eine entsprechend geringe Breite.

Weiterhin kann die Adhäsion der Flüssigkeiten an der Oberfläche des Gehäuses 11 minimiert werden, indem die Oberfläche mit einem Lotuseffekt ausgestattet wird. So perlt Flüssigkeit leichter ab und fließt direkter in den jeweiligen Auffangbehälter.

### Bezugszeichenliste

- 10: Vorrichtung, Dialysegerät
- 11: Gehäuse
- 12: Äußere Komponenten
- 13: Innere Komponenten
- 14: Frontseite, Frontplatte
- 20: Sockel
- 21: Auffangmulde
- 21',21": Wannenfläche
- 22: Aufnahmemittel, Rinne
- 23: Steg
- 30: Sensor
- 40: Abdeckung

## Patentansprüche

1. Vorrichtung (10) zur extrakorporalen Blutbehandlung, umfassend einen extrakorporalen Blutkreislauf zum Pumpen von Blut, wobei sich einzelne Komponenten des extrakorporalen Blutkreislaufs jeweils innerhalb und außerhalb eines Gehäuses (11) der Vorrichtung (10) befinden, und unterhalb des Gehäuses (11) ein Sockel (20) angebracht ist, der eine Auffangmulde (21) aufweist, in der innerhalb des Gehäuses (11) entstehende Leckageflüssigkeiten auffangbar sind, und die Vorrichtung (10) einen Sensor (30) aufweist, mit dem wenigstens das Vorhandensein von Flüssigkeit innerhalb der Auffangmulde (21) messbar ist, und
die Vorrichtung Aufnahmemittel (22) zur Aufnahme von außerhalb des Gehäuses (11) entstehenden Leckageflüssigkeiten aufweist, **dadurch gekennzeichnet, dass** die Aufnahmemittel (22) so ausgebildet sind, dass sie diese Leckageflüssigkeiten ab einer vorgegebenen Menge wenigstens teilweise in die Auffangmulde (21) überführen.

2. Vorrichtung nach Anspruch 1,
**dadurch gekennzeichnet, dass** sie ein Hydrauliksystem zur Bereitstellung von Dialysierflüssigkeit umfasst, wobei sich einzelne Komponenten des Hydrauliksystems jeweils innerhalb und außerhalb eines Gehäuses (11) der Vorrichtung (10) befinden.

3. Vorrichtung nach einem oder beiden der Ansprüche 1 und 2,
**dadurch gekennzeichnet, dass** die Aufnahmemittel (22) von außerhalb des Gehäuses (11) sichtbar und zugänglich sind.

4. Vorrichtung nach Anspruch 2 oder beiden der Ansprüche 2 und 3,
**dadurch gekennzeichnet, dass** das Hydrauliksystem wenigstens einen Vorratsbehälter umfasst, der ein Medium zur Zubereitung des Dialysats enthält, und sich wenigstens der Vorratsbehälter außerhalb des Gehäuses (11) befindet.

5. Vorrichtung nach einem oder mehreren der Ansprüche 1 bis 4,
**dadurch gekennzeichnet, dass** sich der Sockel (20) wenigstens an der Frontseite (14) des Gehäuses (11) über das Gehäuse (11) hinaus erstreckt.

6. Vorrichtung nach Anspruch 5,
**dadurch gekennzeichnet, dass** in dem außerhalb des Gehäuses (11) liegenden Frontbereich des Sockels (20) als Aufnahmemittel wenigstens eine nach oben offene Rinne (22) vorgesehen ist, die eine Verbindung zur Auffangmulde (21) aufweist, wobei die Verbindung zwischen der Rinne (22) und der Auffangmulde (21) so ausgebildet ist, dass in der Rinne (22) gesammelte Leckageflüssigkeiten ab einer vorgegebenen Menge in die Auffangmulde (21) fließen.

7. Vorrichtung nach Anspruch 6,
**dadurch gekennzeichnet, dass** zwischen der Rinne (22) und der Auffangmulde (21) ein Steg (23) angebracht ist, dessen Höhe unterhalb des Rands der Rinne (22) liegt.

8. Vorrichtung nach einem oder mehreren der Ansprüche 5 bis 7,
**dadurch gekennzeichnet, dass** der Sockel (20) im Bereich außerhalb des Gehäuses (11) Mittel zur Zuführung von Leckageflüssigkeiten zu den Aufnahmemitteln (22) aufweist.

9. Vorrichtung nach einem oder mehreren der Ansprüche 5 bis 8,
**dadurch gekennzeichnet, dass** sich die Auffangmulde (21) bis über das Gehäuse (11) hinaus erstreckt und der Sensor (30) in diesem außerhalb des Gehäuses (11) liegenden Bereich der Auffangmulde (21) positioniert ist.

10. Vorrichtung nach einem oder mehreren der Ansprüche 1 bis 9,
**dadurch gekennzeichnet, dass** sich der Sensor (30) am tiefsten Punkt der Auffangmulde (21) befindet.

11. Vorrichtung nach Anspruch 9 oder nach beiden der Ansprüche 9 und 10,
**dadurch gekennzeichnet, dass** der außerhalb des Gehäuses (11) liegende Bereich der Auffangmulde (21) eine abnehmbare Abdeckung (40) aufweist.

12. Vorrichtung nach einem oder mehreren der Ansprüche 1 bis 11,
**dadurch gekennzeichnet, dass** das Gehäuse (11) so ausgebildet ist, dass auβerhalb des Gehäuses (11) entstehende Leckageflüssigkeiten aufgrund einer Kapillarwirkung zu den Aufnahmemitteln (22) geleitet werden.

13. Vorrichtung nach Anspruch 12,
**dadurch gekennzeichnet, dass** die Oberfläche der Frontseite (14) des Gehäuses (11) mehrere eingeprägte Rillen aufweist.

14. Vorrichtung nach einem oder mehreren der Ansprüche 1 bis 13,
**dadurch gekennzeichnet, dass** der Sensor (30) und/oder eine an den Sensor (30) angeschlossene Auswerteinheit der Vorrichtung so ausgebildet sind, dass bei der Erkennung von Flüssigkeiten in der Auffangmulde (21) spezifisch zwischen zwei oder mehr Flüssigkeiten aus der Gruppe Blut, Dialyseflüssigkeit, Konzentrat, Desinfektionsflüssigkeit und Wasser unterschieden werden kann.

15. Vorrichtung nach Anspruch 14,
**dadurch gekennzeichnet, dass** die Unterscheidung der Flüssigkeiten farbselektiv durchführbar ist.

16. Vorrichtung nach einem oder mehreren der Ansprüche 2 bis 15,
**dadurch gekennzeichnet, dass** der extrakorporale Blutkreislauf ein Blutschlauchsystem und das Hydrauliksystem ein Dialyseflüssigkeits-Flusssystem umfasst, und dass die Vorrichtung eine Steuer- und/oder Kontrolleinheit aufweist, durch welche bei Erkennung von Leckagen mittels des Sensors (30) eine automatische Dichtigkeitsüberprüfung des Dialyseflüssigkeits-Flusssystems und/oder des Blutschlauchsystems initiierbar ist.

## Claims

1. Device (10) for extra-corporal blood treatment, including an extra-corporal blood circulation for the pumping of blood, wherein individual components of the extra-corporal blood circulation are located inside and outside a casing (11), respectively, of the device (10), and a base (20) is attached below the casing (11), which includes a collection trough (21), in which leakage fluids occurring within the casing (11) can be collected, and the device (10) including a sensor (30) with which at least the presence of fluid within the collection trough (21) is measurable, and the device including reception means (22) for the reception of leakage fluids occurring outside the casing (11), **characterized in that** the reception means (22) are formed so that, above a specified quantity, they convey at least part of said leakage fluids into the collection trough (21).

2. Device according to claim 1,
**characterized in that** it includes a hydraulic system for the provision of dialysis fluid, wherein individual components of the hydraulic system are inside and outside a casing (11), respectively, of the device (10).

3. Device according to one or both of claims 1 and 2,
**characterized in that** the reception means (22) are visible and accessible from outside the casing (11).

4. Device according to claim 2 or both of claims 2 and 3,
**characterized in that** the hydraulic system includes at least one storage tank which contains a medium for the preparation of the dialysate, and at least the storage tank is located outside the casing (11).

5. Device according to one or more of claims 1 to 4,
**characterized in that** the base (20) extends beyond the casing (11) at least on the front side (14) of the casing (11).

6. Device according to claim 5,
**characterized in that** in the front area of the base (20) located outside the casing (11), as reception means, at least one channel (22) opening towards the top is provided, which comprises a connection to the collection trough (21), wherein the connection between the channel (22) and the collection trough (21) is formed so that, above a specified quantity, leakage fluids collected in the channel (22) flow into the collection trough (21).

7. Device according to claim 6,
**characterized in that** a web (23) is attached between the channel (22) and the collection trough (21), the height whereof lies below the edge of the channel.

8. Device according to one or more of claims 5 to 7,
**characterized in that** the base (20) in the area outside the casing (11) includes means for supplying leakage fluids to the reception means (22).

9. Device according to one or more of claims 5 to 8,
**characterized in that** the collection trough (21) extends up to beyond the casing (11), and the sensor (30) is positioned in this area of the collection trough (21) located outside the casing (11).

10. Device according to one or more of claims 1 to 9,
**characterized in that** the sensor (30) is located at the lowest point of the collection trough (21).

11. Device according to claim 9 or according to both of claims 9 and 10,
**characterized in that** the area of the collection trough (21) located outside the casing (11) comprises a removable cover (40).

12. Device according to one or more of claims 1 to 11,
**characterized in that** the casing (11) is formed so that leakage fluids occurring outside the casing (11) are conducted to the reception means (22) on account of a capillary effect.

13. Device according to claim 12,
**characterized in that** the surface of the front side (14) of the casing (11) includes a plurality of impressed grooves.

14. Device according to one or more of claims 1 to 13,
**characterized in that** the sensor (30) and/or an evaluation unit of the device connected to the sensor (30) are formed so that, in identifying the fluids in the collection trough (21), a specific differentiation between two or more fluids from the group consisting of blood, dialysis fluid, concentrate, disinfecting liquid and water is made possible.

15. Device according to claim 14,
**characterized in that** the differentiation of the liquids is feasible on color-selection basis.

16. Device according to one or more of claims 2 to 15,
**characterized in that** the extra-corporal blood circulation includes blood lines, and the hydraulic system includes a dialysis fluid flow system, and that the device comprises a control and/or monitoring unit, through which, in identifying leakages by way of the sensor (30), an automatic leakage test of the dialysis fluid flow system and/or the blood lines can be initiated.

## Revendications

1. Dispositif (10) permettant le traitement extracorporel du sang, comportant un circuit de sang extracorporel destiné à pomper le sang, différents composants du circuit de sang extracorporel étant disposés respectivement à l'intérieur et à l'extérieur d'un boîtier (11) du dispositif (10), et en dessous du boîtier (11) étant disposé un socle (20) qui comporte une cavité de réception (21), dans laquelle peuvent être collectées les fuites de liquide produites à l'intérieur du boîtier (11), le dispositif (10) comportant un capteur (30) qui permet de mesurer au moins la présence de liquide à l'intérieur de la cavité de réception (21), et le dispositif comportant des moyens de réception (22) permettant de recevoir les fuites de liquide produites à l'extérieur du boîtier (11), **caractérisé en ce que** les moyens de réception (22) sont configurés de telle sorte qu'ils transfèrent les fuites de liquide, à partir d'une quantité prédéfinie, au moins en partie dans la cavité de réception (21).

2. Dispositif selon la revendication 1, **caractérisé en ce que** ledit dispositif comporte un système hydraulique pour la mise à disposition du liquide de dialyse, différents composants du système hydraulique étant disposés à l'intérieur et à l'extérieur d'un boîtier (11) du dispositif (10).

3. Dispositif selon l'une ou les deux revendications 1 et 2, **caractérisé en ce que** les moyens de réception (22) sont visibles et accessibles depuis l'extérieur du boîtier (11).

4. Dispositif selon la revendication 2 ou les deux revendications 2 et 3, **caractérisé en ce que** le système hydraulique comporte au moins un réservoir contenant un produit pour la préparation du dialysat, et **en ce qu'**au moins ledit réservoir est situé à l'extérieur du boîtier (11).

5. Dispositif selon l'une quelconque ou plusieurs des revendications 1 à 4, **caractérisé en ce que** le socle (20) s'étend au moins sur la face frontale (14) du boîtier (11) en s'avançant au-delà du boîtier (11).

6. Dispositif selon la revendication 5, **caractérisé en ce que** dans la zone frontale du socle (20), située en dehors du boîtier (11), il est prévu un moyen de réception formé par au moins une goulotte (22) ouverte vers le haut qui comporte une liaison vers la cavité de réception (21), la liaison entre la goulotte (22) et la cavité de réception (21) étant configurée de telle sorte que les fuites de liquide rassemblées dans la goulotte (22) coulent dans la cavité de réception (21) à partir d'une quantité prédéfinie.

7. Dispositif selon la revendication 6, **caractérisé en ce qu'**entre la goulotte (22) et la cavité de réception (21) est disposée une traverse (23) dont la hauteur se situe au-dessous du bord de la goulotte (22).

8. Dispositif selon l'une quelconque ou plusieurs des revendications 5 à 7, **caractérisé en ce que** le socle (20), dans la zone en dehors du boîtier (11), comporte des moyens permettant d'acheminer les fuites de liquide vers les moyens de réception (22).

9. Dispositif selon l'une quelconque ou plusieurs des revendications 5 à 8, **caractérisé en ce que** la cavité de réception (21) s'étend jusque au-delà du boîtier (11) et **en ce que** le capteur (30) est positionné dans cette zone de la cavité de réception (21), située en dehors du boîtier (11).

10. Dispositif selon l'une quelconque ou plusieurs des revendications 1 à 9, **caractérisé en ce que** le capteur (30) se situe au point le plus bas de la cavité de réception (21).

11. Dispositif selon la revendication 9 ou selon les deux revendications 9 et 10, **caractérisé en ce que** la zone de la cavité de réception (21), située en dehors du boîtier (11), comporte un couvercle (40) amovible.

12. Dispositif selon l'une quelconque ou plusieurs des revendications 1 à 11, **caractérisé en ce que** le boîtier (11) est configuré de telle sorte que les fuites de liquide produites à l'extérieur du boîtier (11) sont guidées vers les moyens de réception (22) en raison d'un effet de capillarité.

13. Dispositif selon la revendication 12, **caractérisé en ce que** la surface de la face frontale (14) du boîtier (11) comporte plusieurs rainures gravées.

14. Dispositif selon l'une quelconque ou plusieurs des revendications 1 à 13, **caractérisé en ce que** le capteur (30) ou une unité d'analyse du dispositif, raccordée au capteur (30), sont configurés de telle sorte que, lors de la détection de liquides dans la cavité de réception (21), une différenciation spécifique entre deux ou plusieurs liquides du groupe comprenant sang, liquide de dialyse, concentrat, liquide de désinfection et eau est possible.

15. Dispositif selon la revendication 14, **caractérisé en ce que** la différenciation des liquides peut être effectuée par une sélection de couleurs.

16. Dispositif selon l'une quelconque ou plusieurs des revendications 2 à 15, **caractérisé en ce que** le circuit de sang extracorporel comporte un système tubulaire pour le sang et le système hydraulique comporte un système d'écoulement du liquide de dialyse, et **en ce que** le dispositif comporte une unité de commande et/ou de contrôle qui, lors de la détection de fuites par le capteur (30), permet d'initier un contrôle d'étanchéité automatique du système d'écoulement du liquide de dialyse et/ou du système tubulaire pour le sang.
